(19) Europäisches Patentamt
European Patent Office
Office européen des brevets

(11) **EP 2 772 189 A1**

(12) **EUROPEAN PATENT APPLICATION**

(43) Date of publication:
**03.09.2014 Bulletin 2014/36**

(51) Int Cl.:
*A61B 5/11* (2006.01)   *A61B 5/00* (2006.01)
*A61B 5/08* (2006.01)   *A61B 5/113* (2006.01)
*G06T 7/00* (2006.01)   *G06T 7/20* (2006.01)

(21) Application number: **13157242.2**

(22) Date of filing: **28.02.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Koninklijke Philips N.V.
5656 AE Eindhoven (NL)**

(72) Inventor: **The designation of the inventor has not yet been filed**

(74) Representative: **Damen, Daniel Martijn
Philips
Intellectual Property & Standards
P.O. Box 220
5600 AE Eindhoven (NL)**

(54) **Apparatus and method for determining vital sign information from a subject**

(57)    The present invention relates to an apparatus (10) for determining vital sign information (R) from a subject (12), comprising a detection unit (22) for detecting radiation (24) from a field of view (42) and for determining characteristic parameter (S) including vital sign information (R) of the subject (12) from different areas (48) of the field of view (42), a frequency analysis unit (30) for determining a spectral parameter of the characteristic parameter (S) derived from the different areas (48), a selection unit (32) for selecting at least one of the areas (48) of the field of view (42) on the basis of the spectral parameter, and a calculation unit (34) for calculating the vital sign information (R) on the basis of the characteristic parameter (S) from the at least one selected area (48).

FIG. 1

EP 2 772 189 A1

## Description

FIELD OF THE INVENTION

The present invention relates to an apparatus for determining vital sign information from a subject and a corresponding method, in particular, the present invention relates to measurements which can be used for remotely determining vital signs of a subject, wherein a region of interest is automatically determined. The present invention is in particular directed to a remote measurement of a respiration rate of a subject.

BACKGROUND OF THE INVENTION

Vital signs of a person, for example the respiration rate or the heart rate serve as indicators for the current state of the person and as predictions of serious medical events. For this reason, vital signs are extensively monitored in inpatient and outpatient care settings, at home or in further health, leisure and fitness settings.

Camera-based monitoring of the vital signs for example the respiration rate or the heart rate is a known technique for fully contactless measuring the vital signs of a person. Since the subject of interest, i.e. the person to be measured can be located freely in the field of view of the camera, the relevant area from which the respective vital sign information should be acquired has to be defined as the input for the expectation of the respective signals. In most applications for contactless vital sign measurements, the region of interest is selected manually or the used camera is directed to the region of interest in advance, however, a movement of the subject leads to incorrect measurements and an impractical use of the system. Therefore, an automatic detection of the region of interest is desired to improve the camera-based monitoring of the vital sign information.

Conventional methods to determine the region of interest for respiration rate or heart rate detection on the basis of contour detection such as face detection are e.g. disclosed in US 2009/0141124 A1. The disadvantage of this method is that the region of interest cannot be detected reliable, if the contour, i.e. the face is partially or fully occluded or hidden when the respective portion of the subject to be measured is covered by a blanket, which is a typical case in hospitals where a monitoring of the respiration rate is critical.

Other methods which are based on a shape analysis, such as chest/thorax detection, for the detection of the region of interest are limited by the position of the subject within the field of view or by the worn clothing so that those detection methods are less reliable.

A method for identifying a region of interest for respiration monitoring is for example known from EP 0 919 184 A1, whereas the region of interest is determined on the basis of changed portions between successive images captured from the field of view, wherein the changes between successive images can be based on disturbance signals, which do not refer to vital signs. Hence, the method known from this document is less reliable.

A further method for monitoring the respiration of a subject is known from US 7,431,700 B2, wherein a time based change in the image data is analyzed and a periodic appearance is detected as respiration, however, since all time based changes in the whole field of view are considered and no region of interest is detected, the presence of disturbing signals can lead to incorrect measurements of the respiration rate. Hence, the method from this document is less reliable and has an increased technical effort.

The disadvantage of the known methods to detect a region of interest for remotely detection vital sign information from a subject is that the whole image detected from the field of view is used to detect the vital sign information so that these methods are susceptible to disturbance signals in the field of view and to movements of the subject within the field of view so that the known methods for determining vital signs from the subject are less reliable.

SUMMARY OF THE INVENTION

It is an object of the present invention to provide an improved apparatus and a corresponding improved method for determining vital sign information from a subject which is less susceptible to disturbing signals and movements of the subject to be measured and provides in general a higher reliability regarding the vital sign detection.

According to one aspect of the present invention, an apparatus for determining vital sign information from a subject is provided, comprising:

- a detection unit for detecting radiation from a field of view and for determining characteristic parameter including vital sign information of the subject from different areas of the field of view,
- a frequency analysis unit for determining a spectral parameter of the characteristic parameter derived from the different areas,
- a selection unit for selecting at least one of the areas of the field of view on the basis of the spectral parameter, and
- a calculation unit for calculating the vital sign information on the basis of the characteristic parameter from the at least one selected area.

According to another aspect of the present invention, a method for determining vital sign information from a subject is provided, comprising the steps of:

- detecting radiation from a field of view,
- determining characteristic parameter including vital sign information of the subject from different areas of the field of view,

- determining a spectral parameter from the characteristic parameter derived from the different areas,
- selecting at least one area of the field of view on the basis of the spectral parameter, and
- calculating the vital sign information on the basis of the characteristic parameter from the at least one selected area.

According to still another aspect of the present invention, a computer program is provided comprising program code means for causing a computer to carry out the steps of the method according to the present invention when said computer program is carried out on a computer.

Preferred embodiments of the present invention are defined in the dependent claims. It shall be understood that the claimed method has similar and/or identical preferred embodiments as the claimed device and as defined in the dependent claims.

The present invention is based on the idea to analyze different areas of the field of view and to derive a characteristic parameter from the different areas of the field of view. A spectral analysis is performed on this characteristic parameter in order to determine whether the characteristic parameter comprises vital sign information or whether the characteristic parameter merely includes disturbance signals or high frequency noise. On the basis of this spectral analysis, those areas of the field of view can be selected which provide a characteristic parameter including vital sign information so that the vital sign information can be calculated on the basis of the areas which provide these vital sign information. Hence, the region of interest can be determined from the field of view continuously even if the subject to be measured is moving within the field of view or if the indicative portions to be measured are partially obstructed so that the determination of the vital sign information is in general more reliable.

In a preferred embodiment, the selection unit is adapted to select a plurality of different areas of the field of view on the basis of the spectral parameter. If the calculation is performed on the basis of a plurality of different areas of the field of view, the calculation is less susceptible for disturbance signals and more robust, since the vital sign information is derived from different portions of the subject.

In a preferred embodiment, the detection unit is an image detection unit for providing image data from the field of view. This is a simple solution to provide a remote detection for determining the vital sign information from the subject.

In a preferred embodiment, the images or the image frames captured by the image detection unit are divided sectional in order to define the different areas. This allows a sectional computation of the vital sign information over the different areas or blocks of the images or image frames.

In a preferred embodiment, the detection unit is adapted to determine motion vectors as the characteristic parameter corresponding to the vital sign information from the different areas on the basis of pattern detection of the detected image data. This is a practical solution to determine the respiration rate of the subject, since the motion vectors correspond to the movement of indicative portions of the subject and the respective pattern can be easily determined on the basis of the captured image data.

In a preferred embodiment, the detection unit is adapted to determine alternating signals as the characteristic parameter on the basis of pattern detection of the detected image data. This is a simple possibility to determine signals which can be easily analyzed in order to identify areas including vital sign information.

In a preferred embodiment, the calculation unit is adapted to calculate the alternating signal on the basis of the motion vectors determined from the at least one selected area. This is a practical solution to determine a signal from the detected motion vectors which can be analyzed with low technical effort.

In a preferred embodiment, the spectral parameter is a spectral energy distribution of the characteristic parameter. This is a possibility to determine whether vital sign information is included in the characteristic parameter with low technical effort.

In a preferred embodiment, the selection unit is adapted to select the at least one area of the field of view if the spectral energy of a predefined frequency band of the respective characteristic parameter exceeds a predefined threshold level. This is a reliable possibility to determine whether the at least one area of the field of view comprises vital sign information, since the frequency spectral of the vital sign information has a characteristic frequency band distinguishing from disturbing signals and from noise.

In a preferred embodiment, the selection unit is adapted to determine a weight factor for each of the selected different areas and wherein the calculation unit is adapted to calculate the vital sign information on the basis of the characteristic parameter of the selected area weight by means of the respective weight factor. This is a practical solution to determine the vital sign information considering the quality of the detected characteristic parameter so that the calculation results become more reliable.

In a further preferred embodiment, the selection unit is adapted to perform the selection on a regular basis and wherein the weight factor for each of the selected area is determined on the basis of a frequency of selection of the respective areas. This is a simple solution to determine the weight factor for each of the selected areas with low technical effort. The different areas are processed separately and frequently, wherein the signals derived from the different areas are weighted by the weight factor which is dependent on the number of times the respective area has been selected, wherein more weight is given to the signals derived from those areas which were selected more often. In other words, the signals of the different areas are weight by accumulating the results of the frequency analysis.

In a preferred embodiment, the motion vectors of the different areas are determined on a regular basis and stored in a storage device. This is a possibility to perform a detailed analysis of the motion vector, since the respective vectors are stored in a storage device and can be weighted and evaluated in one step.

It is preferred if the storage device is a shift register storing a predefined amount of motion vectors. In this embodiment, the oldest motion vectors are removed and all subsequent vectors are shifted in the register to make space for the latest vector. This allows the calculation of the weight factors continuously so that the vectors can be easily weighted by the weight factor.

In a preferred embodiment, a general motion vector is calculated on the basis of the motion vectors determined from the selected areas and weighted on the basis of weight factor for the respective area. This is a reliable possibility to determine a precise motion vector derived from the field of view.

It is preferred if the calculation unit is adapted to calculate the vital sign information on the basis of the general motion vector. This is an overall solution in order to determine a precise and reliable vital sign information from the subject in the field of view.

As mentioned above, the present invention provides an apparatus and a method which determine the signals received from different areas or sections of the field of view and to evaluate the received signals independently so that the region of interest for the remote measurement of the vital sign information can be determined with a high reliability on the basis of the real vital sign information and can be adapted continuously. Since the region of interest is defined in the field of view without defining a certain region and without a priority of a location or a size of the region of interest, the source of the signals to be evaluated can be selected freely and the reliability and the preciseness of the calculated vital sign information is increased. Further, due to the frequency analysis of the signals received from the different areas or sections of the field of view and due to the calculated weight factors depending on the frequency analysis, the calculated vital sign information is robust against disturbance signals, noise and movement of the subject.

Hence, the vital sign information can be determined from a subject with high reliability and high preciseness on the basis of a remote measurement technique.

BRIEF DESCRIPTION OF THE DRAWINGS

These and other aspects of the invention will be apparent from and elucidated with reference to the embodiments described hereinafter. In the following drawings

Fig. 1 shows a schematic illustration of a general layout of an apparatus for determining vital sign information from a subject,
Fig. 2 shows a schematic illustration of a subject's motion indicative of an exemplary vital sign information,
Fig. 3 shows a timing diagram of an alternating signal derived from the subject corresponding to the vital sign information,
Fig. 4 shows a frequency diagram of the alternating signal shown in fig. 3,
Figs. 5a-e show a schematic image sequence for illustrating the selection of different image sections in the field of view for calculating the vital sign information,
Fig. 6 shows a schematic block diagram representing the steps of an embodiment of a method for determining vital sign information from a subject, and
Fig. 7 shows a schematic timing diagram of a vital signal derived from the region of interest of the field of view.

DETAILED DESCRIPTION OF THE INVENTION

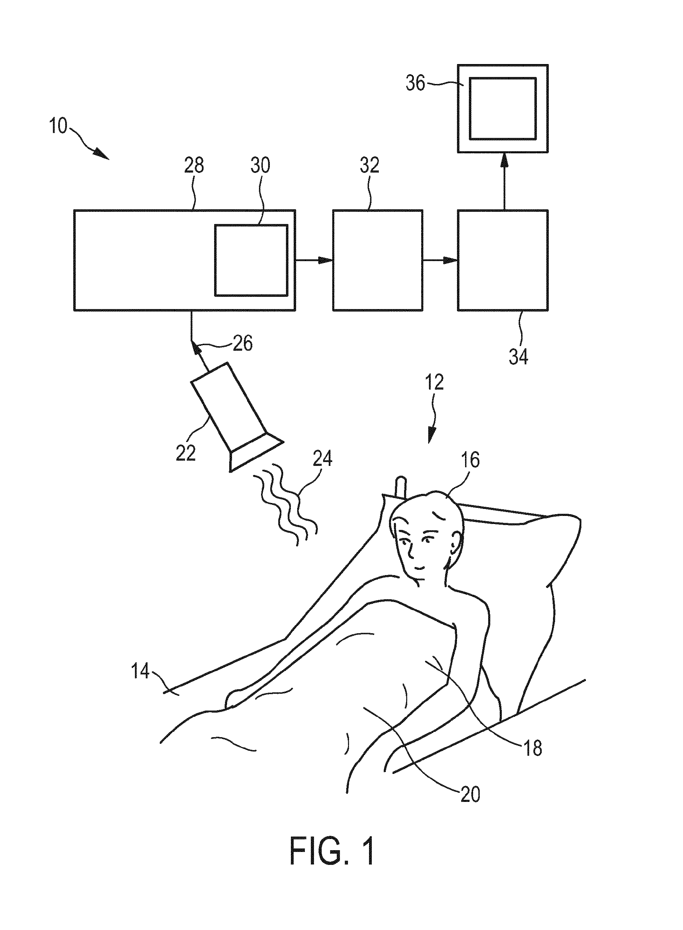

Fig. 1 shows a schematic drawing of an apparatus generally denoted by 10 for determining vital sign information from a subject 12. The subject 12, e.g. a patient staying in bed, is resting on a support 14. The subject's head 16 is usually a non-indicative portion regarding the respiration of the subject 12, wherein an indicative portion, the chest 18 or the belly 18 is covered by a blanket 20. The general problem of the common situation shown in fig. 1 is that a signal derived from the motion of the chest 18 as indicative portion regarding the respiration is attenuated and the signal detection on the basis of a remote measurement is considerably difficult.

The apparatus 10 comprises an image detection device 22, e.g. a monochromatic camera which can be used for recording image frames of the subject 12. The image frames can be derived from electromagnetic radiation 24 emitted or reflected by the subject 12. For extracting the image information from the image data 26, e.g. a sequence of image frames, the image data 26 is provided to an image processing unit 28.

The image detection device 22 is adapted to capture images belonging to at least a spectral component of the electromagnetic radiation 24. The image detection device 22 may provide continuous image data or a discrete sequence of image frames captured from a field of view including the subject 12 to be measured.

The image processing unit 28 is adapted to evaluate the image data 26 in general and to divide the captured images in sections or areas of the field of view and to evaluate the image sections separately in order to determine a region of interest. The image processing unit 28 divides the captured images into the sections or areas and detects motion vectors from the different sections corresponding to motions of objects in the field of view including the motion of the subject 12 and in particular the motion of the chest 18 as indicative portion of the respiration. The motion vectors are determined by means of pattern detection in the image sections or by means of edge detection in the image sections. A method for edge or pattern detection and for deriving the motion vectors from the captured image frames is for example disclosed by WO 2012/140531 A1.

The image processing unit 28 determines an alternating signal from the motion vectors and determines a spectral parameter of the alternating signal by means of a frequency analysis unit 30 as described in detail in the following. The spectral parameter of each of the sections of the image data 26 are provided to a selection unit 32. The selection unit 32 selects the sections of the image data 26 from which an alternating signal is derived including vital sign information from the subject 12. The selection unit 32 selects the sections on the basis of the respective spectral parameter. The spectral parameter is a frequency spectrum or a spectral energy distribution of the alternating signal. Since the vital sign information has a characteristic spectral energy distribution or a characteristic frequency, the selection unit can select the sections which comprise vital sign information of the subject 12. The selection unit 32 determines a weight factor for each of the different image sections dependent on the frequency analysis. The weight factor is determined corresponding to a frequency how often the respective image section has been selected. The selection unit 32 provides the selection information including the weight factors to a calculation unit 34 which is adapted to calculate the vital sign information on the basis of the motion vectors derived from each of the selected sections. By means of the weight factor, the calculation unit 34 weights the motions vectors of the respective image sections, wherein more weight is given to the sections having a spectral parameter indicating a better quality of the received motion vector, i.e. the sections which have been selected more often. Those image sections which are selected less often or never due to a high disturbing signal amount are less weight or removed. The calculation unit 34 provides the calculated vital sign information to an output device 36 such as a monitor.

Hence, the apparatus 10 determines those sections of the captured image frames which contain the respiration motion and which can be used for defining the region of interest for determining the respiration of the subject 12. The image processing unit 28 determines the motion data from the sections of the image data 26 and the selection unit 32 selects those sections which contain vital sign information and does not select those sections which contain disturbance signals or noise.

For determining the region of interest, a plurality of image frames is captured and evaluated and the weight factor (called persistence) is determined corresponding to the number of times each section is selected as vital sign containing section. Finally, the motion vectors from each of the selected sections are weighted by the weight factor to determine a general motion vector and to determine the vital sign information e.g. the respiration rate as described in the following.

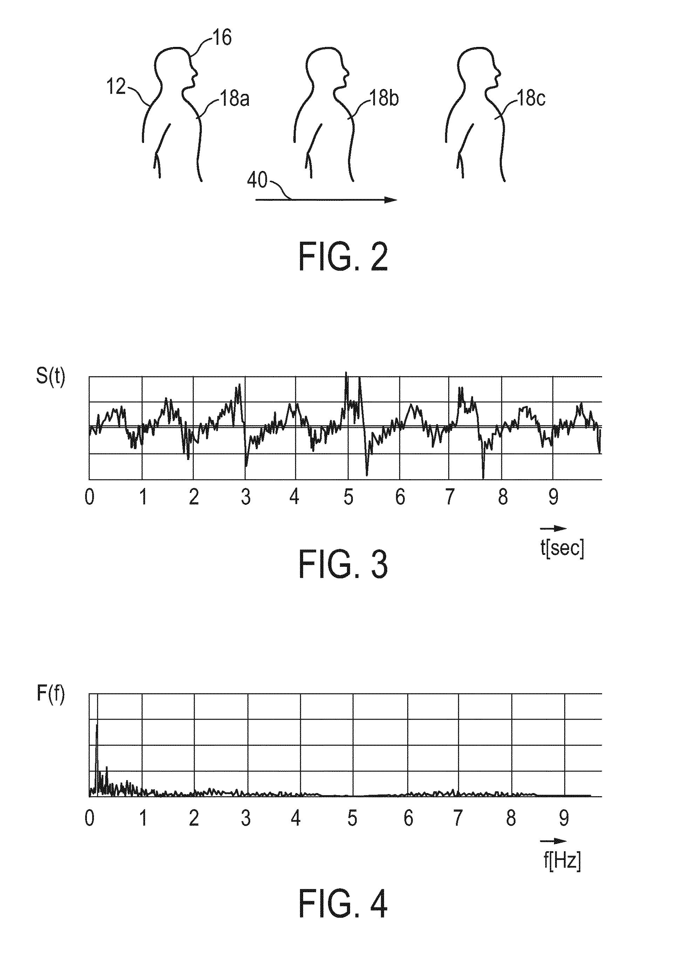

Fig. 2 shows a schematic illustration of the subject 12 in order to describe the remote measurement of the respiration of the subject 12. The subject 12 undergoes a characteristic motion of an indicative portion 18 (the chest 18) due to the respiration. When breathing, expansion and contraction of the lung's courses slight motion of characteristic portions of liven beings, e.g. lifting and lowering of the chest 18. Also, abdominal breathing can course characteristic motion of respective parts of the subject's body 12. At least partially periodic motion patterns included by physiological processes can occur in many living beings, particularly in human beings or animals.

Over time, as indicated by an arrow 40, the indicative portion 18 is moved between a contracted position, indicated by reference numerals 18a, 18c, and an extracted portion, indicated by 18b. Essentially, based on this motion pattern, for instance the respiration rate or respiration rate variability can be assessed by means of pattern or edge detection in a captured image sequence. While the indicative portion 18 is pulsating over time, the head 16 as a non-indicative portion 16 remains substantially motionless.

Certainly, also the head 16 undergoes diverse motion over time. However, these motions do not correspond to the periodic pulsation of the chest 18 and can be distinguished by means of the frequency analysis.

Fig. 3 shows a timing diagram of an alternating signal derived from the movement pattern and/or from motion vectors of the different image sections which can be for example determined on the basis of a frame or an edge detection in the respective image section. The alternating signal is generally denoted by S(t). The alternating signal S in this particular case corresponds to the movement of the chest 18 of the subject 12 derived from an image section corresponding to the image data received from the respective indicative portion 18. The alternating signal S shows a characteristic variation corresponding to the movement of the chest 18 i.e. the breathing rate of the subject 12. The alternating signal S also shows a high-frequency noise superimposed to the breathing rate.

The alternating signals S are derived from each of the image sections of the field of view wherein a plurality of image sections comprise vital sign information such as a breathing rate and many image sections may comprise disturbing signals which are not related to vital sign information of the subject 12 or other alternating signals which comprise mostly high-frequency noise. In order to identify those image sections from which vital sign information can be derived, the analysis unit 28 comprises the frequency analysis device 30 to perform a frequency analysis of the alternating signals. The frequency analysis is preferably performed by filtering the alternating signals S and/or by performing a Fourier Transformation, in particular a Fast Fourier Transformation (FFT) of the alternating signal S. From the alternating signal, a frequency spectrum is derived in order to identify the image section including vital sign information as described in the following.

Fig. 4 shows a frequency spectrum of the alternating signal S shown in Fig. 3 generally denoted by F(f). The frequency spectrum F shows a large frequency component in a low frequency band, in this particular case between 0 and 1 Hertz, which correspond to the breathing rate of an adult which is normally not higher than 1 Hertz, i.e. 60 breathes per minute. The frequency components higher than a predefined frequency band, e.g. 1 Hertz for adults and 2 Hertz for infants are usually disturbing signals in the image data 26 or correspond to noise of the alternating signal S. In order to characterize the quality of the alternating signal S, the spectral energy of the alternating signal S is determined and an image section is defined as an image section including vital sign information if the spectral energy of the alternating signal S in a predefined frequency band exceeds a predefined threshold level or exceeds a percentage of spectral energy compared to a second frequency band, e.g. the whole frequency spectrum. E.g. if the spectral energy between 0 and 1 or 2 Hertz is larger than a predefined threshold level, e.g. larger than 50 % of the entire spectral energy of the alternating signal S or a predefined range of the spectrum, e.g. 2 ... 3 Hz, 3 ... 4 Hz, ... On the basis of the spectral energy, the image sections are evaluated to select the image sections in the field of view and to determine the region of interest as described in the following.

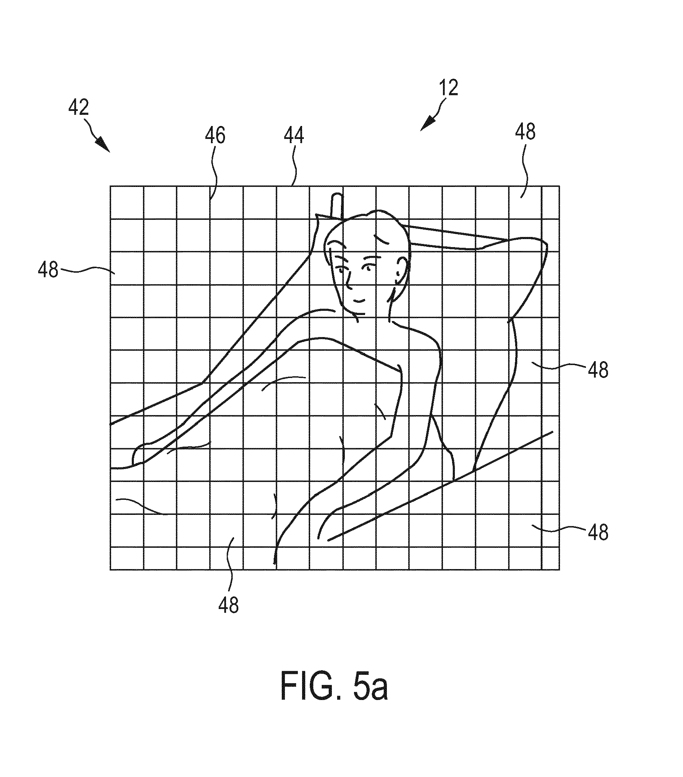

Figs. 5a-e show a schematic image from a field of view for explaining the detection of the vital sign information from the subject 12 on the basis of detected image data 26.

The field of view detected by the image detection device 22 shown in fig. 5a-e is generally denoted by 42. An image frame 44 representing the field of view 42, which is captured by the image detection device 22, shows the subject 12 which is in this case a human being to be measured.

In the image frame 44, a grid 46 divides the image frame 44 in different portions and defines different image sections 48 to distinguish different areas in the field of view 42 and to determine different motion vectors in the field of view 42.

First, movement pattern are derived from each of the image sections 48 of the image frame 44 and the alternating signals S are determined from motion vectors determined from the movement pattern of each of the image sections 48 as described above. The motion vectors are determined by pattern detection or edge detection within the different image sections 48. On the basis of the frequency analysis performed by the frequency analysis unit 30, it is determined whether the movement pattern of the different image sections 48 corresponds to vital sign information in the field of view 42 or whether the movement pattern are disturbance signals or noise.

The determination whether the movement pattern include vital sign information or not is performed as described above on the basis of the spectral parameter and/or the spectral energy and whether the spectral energy in a predefined frequency band is larger than a predefined percentage of the entire spectral energy of the respective alternating signal.

On the basis of these data, which are determined for each of the image sections 48, the selection unit 32 selects those image sections 48 which include the vital sign information.

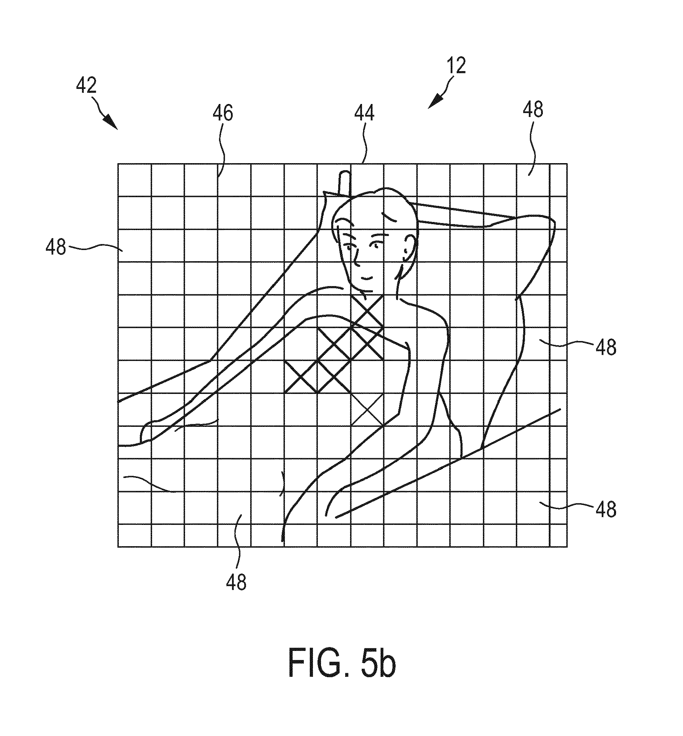

An example for the selected image sections 48 is schematically shown in fig. 5b, wherein the selected image sections 48 are marked by means of a cross.

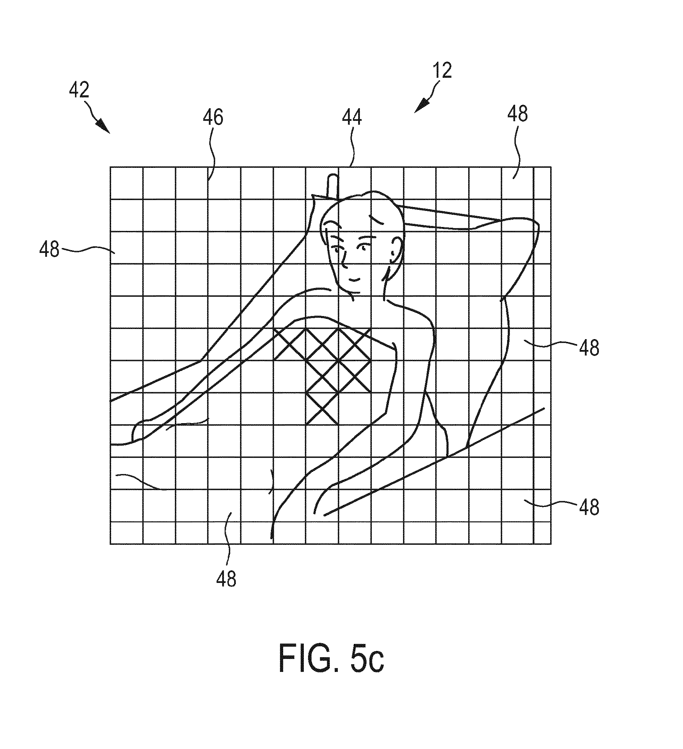

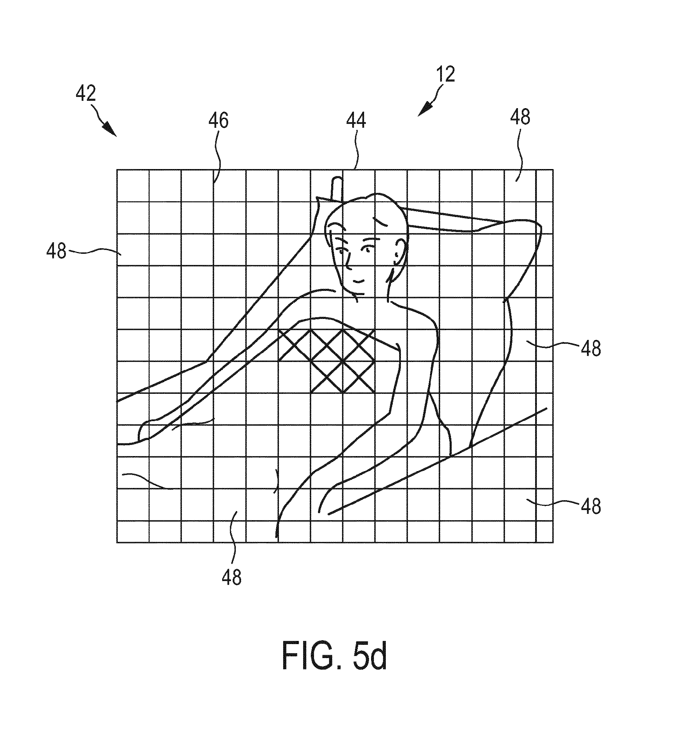

The process of capturing image frames 44 and determining the alternating signals S from each of the image sections 48 and the selection of the image sections 48 including vital sign information is performed on a regular basis or frequently and different image sections 48 may be selected in a predefined time frame or in consecutive image frames 44. Two further image frames 44 including the respectively selected image sections are shown as an example in fig. 5c and fig. 5d.

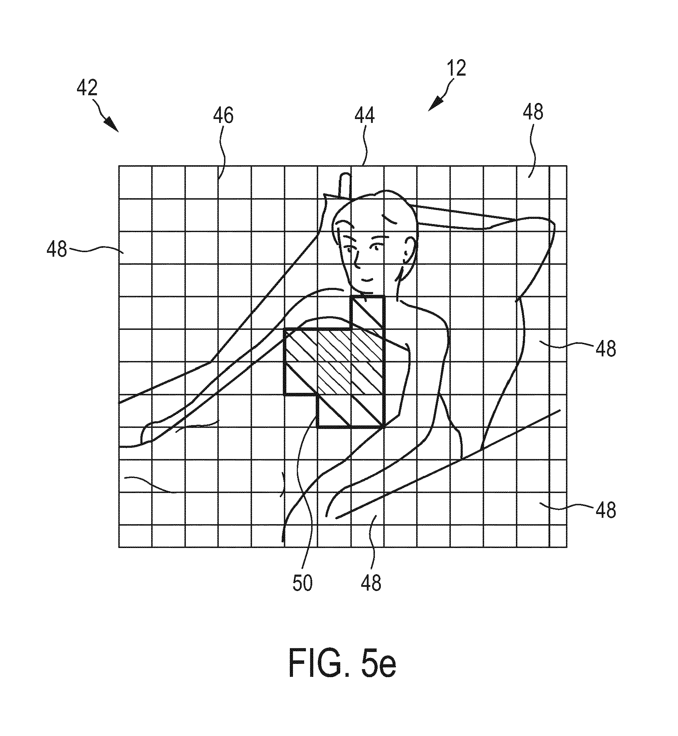

From the selected image sections 48, a region of interest 50 is determined as shown in fig. 5e. In the region of interest 50, the respectively selected image sections 48 are characterized by different hatching corresponding to the number of times the respective image sections 48 have been selected as shown in figs. 5b, 5c and 5d.

The motion vectors which are derived from the sections 48 of the region of interest 50 are weighted by the weight factor (persistence) which is determined on the basis of the frequency or the number of times the respective section 48 has been selected in a predefined time frame.

The region of interest 50 allows a reliable and robust measurement since all sections 48 which are part of the region of interest 50 are weighted over time. The weight factor is proportional to the number of times the respective section is selected.

The respective weight factor $W_i$ for each section 48 is calculated by means of the formula:

$$W_i = \frac{N_i}{n}$$

wherein $N_i$ is the number of times the respective section 48 has been selected, n is the number of frames considered to calculate the motion vectors and i corresponds to the respective section 48.

The motion vectors are preferably stored in a memory and a general motion vector G is calculated after a predefined time frame by means of the formula:

$$\vec{G} = \sum_i W_i * \vec{M}_i$$

wherein $W_i$ is the weight factor of the respective section 40 and $M_i$ is the motion vector of the respective section 48. Hence, the motion vectors $M_i$ of the region of interest 50 are weighted by means of the frequency the respective section 48 has been selected to calculate the general motion vector G.

Preferably, the memory for storing the motion vectors is a shift register wherein the motion vectors $M_i$ of each image frame 44 and for each section 48 is buffered. The oldest motion vector $M_i$ per section 48 is removed and all subsequent vectors $M_i$ are shifted in the shift register. Each motion vector $M_i$ is than weighted by means of the weight factor corresponding to the frequency the respective section 48 has been selected. I.e. if a section 48 has not been selected, the weight factor is 0 and if a section 48 has been selected in each of the image frames 44, the weight factor is 1.

Hence, the vital sign information can be calculated with high precision on the basis of the respective quality of the signal received from the respective indicative portion 18 of the subject 12.

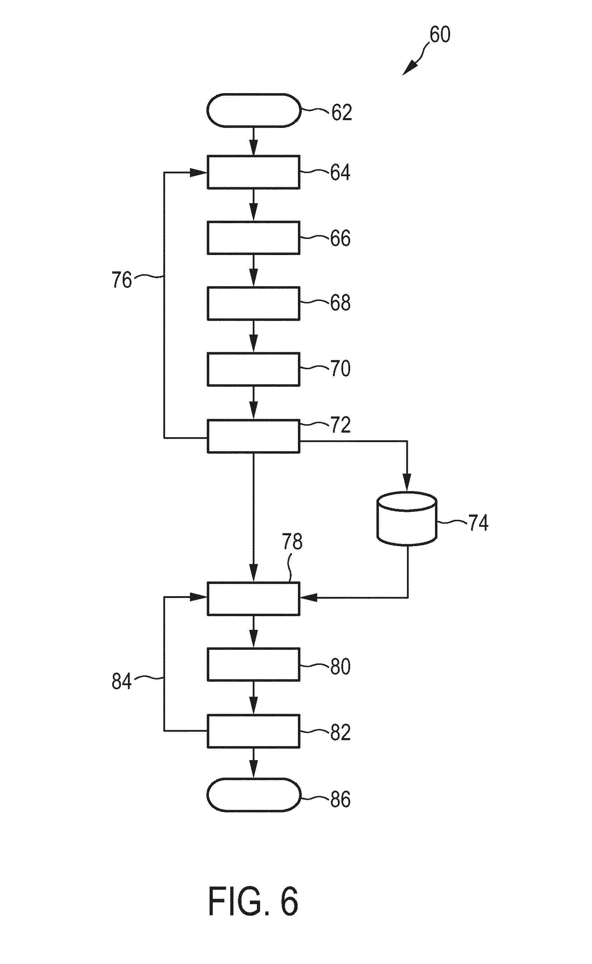

Fig. 6 shows a block diagram illustrating method steps to detect the vital sign information from the subject 12 and to calculate the vital sign information. The method is generally denoted by 60. The method 60 starts with step 62. At 64, an image frame 44 is detected by means of the image detection device 22.

The image frame 44 is evaluated at step 66 by the image processing unit 28 by means of pattern detection or edge detection and the motion vectors $M_i$ are determined for each of the image sections 48 as described above. Depending on the motion vectors $M_i$, a corresponding alternating signal S is calculated for each of the image sections 48.

At step 68, the alternating signals S are analyzed by means of the frequency analysis unit 30 in order to determine whether vital sign information is included in the motion vector.

At step 70, the image sections 48 which fulfill the respective criteria are selected, i.e. those image sections 48 are selected which have a spectral parameter or a spectral energy larger than the predefined threshold level.

At step 72, the motion vectors $M_i$ for each of the image sections 48 and the information which of the image sections 48 has been selected in this image frame 44, are stored in a memory 74, which is preferably a shift register.

The steps 64 to 72 are repeated as indicated by a feedback loop 76 in order to capture and evaluate frequently new image frames 44.

At step 78, the weight factors $W_i$ are calculated

on the basis of the data stored in the memory 74.

At step 80, the general motion vector G is calculated on the basis of the motion vectors $M_i$ for each of the image frames 48 and the weight factors $W_i$ as described above. Finally, the vital sign information is calculated on the basis of the general motion vector G as shown at step 82. The calculation of the vital sign information is performed on a regular basis as indicated by a feedback loop 84.

The method 60 ends at step 86.

Hence, the vital sign information can be calculated continuously on the basis of the frequently captured image frames 44 and the respective detection steps.

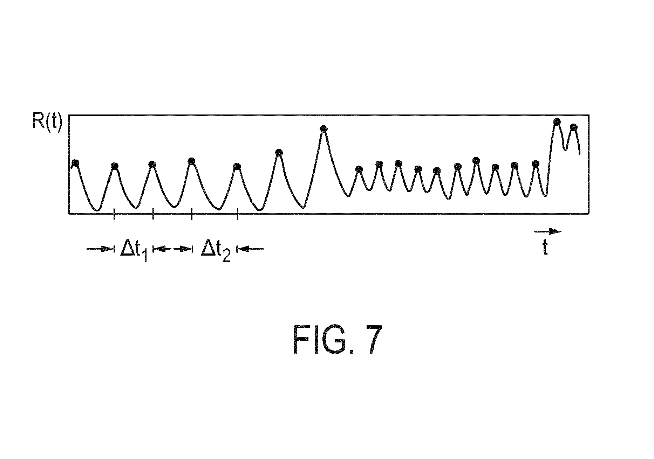

Fig. 7 shows a timing diagram of a vital sign information derived from the general motion vector G and generally denoted by R(t). The vital sign information R(t) is in this particular case a respiration signal derived from the motion of the chest 18 of the subject 12. From the so determined respiration signal R, the respiration rate can be detected on the basis of the maxima of the respiration signal R as indicated by dots in fig. 7. Time distances between the dots are shown in fig. 7 as an example by $\Delta t1$ und $\Delta t2$. The respiration rate is calculated by means of the reciprocal value of the time distance $\Delta t1$, $\Delta t2$ between the maxima in the respiration signal R or an average of the time distances shown in fig. 7.

Hence, the respiration rate can be easily derived from the motion vector G and since the region of interest 50 is automatically determined on the basis of the movement of the chest 18 of the subject 12 and weighted by the weight factor W, the respiration rate can be determined from the image data 26 with high reliability and high preciseness.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Apparatus (10) for determining vital sign information (R) from a subject (12), comprising:

    - a detection unit (22) for detecting radiation (24) from a field of view (42) and for determining characteristic parameter (S) including vital sign information (R) of the subject (12) from different areas (48) of the field of view (42),
    - a frequency analysis unit (30) for determining a spectral parameter of the characteristic parameter (S) derived from the different areas (48),
    - a selection unit (32) for selecting at least one of the areas (48) of the field of view (42) on the basis of the spectral parameter, and
    - a calculation unit (34) for calculating the vital sign information (R) on the basis of the characteristic parameter (S) from the at least one selected area (48).

2. Apparatus as claimed in claim 1, wherein the selection unit (32) is adapted to select a plurality of different areas (48) of the field of view (42) on the basis of the spectral parameter.

3. Apparatus as claimed in claim 1 or 2, wherein the detection unit (22) is an image detection unit (22) for providing image data (26) from the field of view (42).

4. Apparatus as claimed in claim 3, wherein the detection unit (22) is adapted to determine motion vectors (M) as the characteristic parameter (S) corresponding to the vital sign information (R) from the different areas (48) on the basis of pattern detection of the detected image data (26).

5. Apparatus as claimed in claim 3, wherein the detection unit (22) is adapted to determine alternating signals (S) as the characteristic parameter (S) on the basis of pattern detection of the detected image data (26).

6. Apparatus as claimed in claim 4 or 5, wherein the calculation unit (34) is adapted to calculate the alternating signal (S) on the basis of the motion vectors (M) determined from the at least one selected area (48).

7. Apparatus as claimed in claim 1 or 2, wherein the spectral parameter is a spectral energy distribution of the characteristic parameter.

8. Apparatus as claimed in claim 7, wherein the selection unit (32) is adapted to select the at least one area (48) of the field of view (42) if the spectral energy of a predefined frequency band of the respective characteristic parameter (S) exceeds a predefined threshold level.

9. Apparatus as claimed in claim 2, wherein the selection unit (32) is adapted to determine a weight factor (W) for each of the selected different areas (48) and wherein the calculation unit (34) is adapted to calculate the vital sign information (R) on the basis of the characteristic parameter (S) of the selected area (48) weight by means of the respective weight factor (W).

10. Apparatus as claimed in claim 9, wherein the selection unit (32) is adapted to perform the selection on a regular basis and wherein the weight factor (W) for each of the selected areas (48) is determined on the basis of a frequency (n) of selection of the respective areas (48).

11. Apparatus as claimed in claim 5, wherein the motion vectors (M) of the different areas (48) are determined on a regular basis and stored in a storage device (74).

12. Apparatus as claimed in claim 11, wherein the storage device (74) is a shift register (74) storing a predefined amount of motion vectors (M).

13. Apparatus as claimed in any of claims 4 to 9, wherein a general motion vector (G) is calculated on the basis of the motion vectors (M) determined from the selected areas (48) and weighted on the basis of the weight factor (W) of the respective area (48).

14. Apparatus as claimed in claim 13, wherein the calculation unit (34) is adapted to calculate the vital sign information (R) on the basis of the general motion vector (G).

15. Method (60) for determining vital sign information (R) from a subject (12), comprising the steps of:

    - detecting (64) radiation (24) from a field of view (42),
    - determining (66) characteristic parameter (S) including vital sign information (R) of the subject (12) from different areas (48) of the field of view (42),
    - determining (68) a spectral parameter from the characteristic parameter (S) derived from the different areas (48),
    - selecting (70) at least one area (48) of the field of view (42) on the basis of the spectral parameter, and
    - calculating (82) the vital sign information (R) on the basis of the characteristic parameter (S) from the at least one selected area (48).

FIG. 1

FIG. 2

FIG. 3

FIG. 4

FIG. 5a

FIG. 5b

FIG. 5c

FIG. 5d

FIG. 5e

FIG. 6

FIG. 7

Europäisches
Patentamt

European
Patent Office

Office européen
des brevets

**EUROPEAN SEARCH REPORT**

Application Number

EP 13 15 7242

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/100593 A1 (KONINKL PHILIPS ELECTRONICS NV [NL]; CENNINI GIOVANNI [NL]; JEANNE VIN) 10 September 2010 (2010-09-10) | 1-3,5,15 | INV.<br>A61B5/11<br>A61B5/00<br>A61B5/08 |
| Y | * abstract * <br> * page 10, line 15 - line 18 * <br> * page 13, line 25 - page 15, line 2 * <br> * figures 1, 2, 4A, 4B * | 4,6-14 | A61B5/113<br>G06T7/00<br>G06T7/20 |
| X | EP 1 645 840 A1 (SUMITOMO OSAKA CEMENT CO LTD [JP]; UNIV KEIO [JP] SUMITOMO OSAKA CEMEN) 12 April 2006 (2006-04-12) | 1-3,5,15 | |
| Y | * figures 2, 8-10 * <br> * paragraph [0056] - paragraph [0059] * <br> * paragraph [0071] - paragraph [0079] * | 4,6-14 | |
| Y | KAZUKI NAKAJIMA ET AL: "A METHOD FOR MEASURING RESPIRATION AND PHYSICAL ACTMTY IN BED BY OPTICAL FLOW ANALYSIS", 19TH INTERNATIONAL CONFERENCE ON ENGINEERING IN MEDICINE AND BIOLOGY SOCIETY, vol. 5, 30 October 1997 (1997-10-30), pages 2054-2057, XP055067785, * figures 1-4 * <br> * page 2054 - page 2056 * | 4,6-8 | TECHNICAL FIELDS SEARCHED (IPC)<br>A61B<br>G06T |
| Y | EP 2 390 840 A2 (SNELL LTD [GB]) 30 November 2011 (2011-11-30) <br> * paragraph [0023] - paragraph [0026] * <br> * paragraph [0058] * <br> * paragraph [0138] - paragraph [0139] * | 4,6,9-14 | |

The present search report has been drawn up for all claims

| Place of search | Date of completion of the search | Examiner |
|---|---|---|
| The Hague | 5 July 2013 | Kowalczyk, Szczepan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                     EP 13 15 7242

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2013

| Patent document cited in search report | | Publication date | Patent family member(s) | | Publication date |
|---|---|---|---|---|---|
| WO 2010100593 | A1 | 10-09-2010 | CN | 102341811 A | 01-02-2012 |
| | | | EP | 2404261 A1 | 11-01-2012 |
| | | | JP | 2012519527 A | 30-08-2012 |
| | | | US | 2011311143 A1 | 22-12-2011 |
| | | | WO | 2010100593 A1 | 10-09-2010 |
| EP 1645840 | A1 | 12-04-2006 | AU | 2004245814 A1 | 16-12-2004 |
| | | | CA | 2528822 A1 | 16-12-2004 |
| | | | EP | 1645840 A1 | 12-04-2006 |
| | | | JP | 3764949 B2 | 12-04-2006 |
| | | | JP | 2005003366 A | 06-01-2005 |
| | | | US | 2006279428 A1 | 14-12-2006 |
| | | | WO | 2004109227 A1 | 16-12-2004 |
| EP 2390840 | A2 | 30-11-2011 | EP | 1984893 A2 | 29-10-2008 |
| | | | EP | 2077525 A1 | 08-07-2009 |
| | | | EP | 2390840 A2 | 30-11-2011 |
| | | | KR | 20090006068 A | 14-01-2009 |
| | | | US | 2009153730 A1 | 18-06-2009 |
| | | | WO | 2007093780 A2 | 23-08-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090141124 A1
- EP 0919184 A1
- US 7431700 B2
- WO 2012140531 A1